# DEMANDE DE BREVET EUROPEEN

(11) **EP 1 221 308 A1**
(43) Date de publication de la demande: **10.07.2002**
(21) Numéro de dépôt: 02290014.6
(22) Date de dépôt: 07.01.2002
(51) Int. Cl.: A61B 17/70

(54) **Dispositif d'ostéosynthèse pour colonne vertrébrale**

(30) Priorité: 09.01.2001 FR 0100206
(71) Demandeur: Edouard, Brice, 75016 Paris (FR)
(72) Inventeur: Edouard, Brice, 75016 Paris (FR)
(74) Mandataire: Le Bras, Hervé

(57) **Abrégé**

L'invention concerne un dispositif d'ostéosynthèse pour colonne vertébrale, comportant au moins deux organes d'ancrage (3) adaptés à être fixés à deux vertèbres consécutives de la colonne vertébrale, et un organe de liaison (2) adapté à relier les deux organes d'ancrage (3) en exerçant des contraintes à l'encontre du rapprochement des deux organes d'ancrage (3) en translation l'un vers l'autre, l'organe de liaison (2) étant déformable élastiquement en flexion autour d'au moins un axe de déformation, caractérisé par le fait que l'organe de liaison est constitué d'une plaque (2) courbée dans le sens antéro-postérieur pour s'adapter à la courbure de la colonne vertébrale, et comporte des moyens d'immobilisation (6, 8) de chaque organe d'ancrage (3), et par le fait que ladite plaque (2) présente entre deux moyens d'immobilisation, au moins une fente (15) transversale susceptible d'assurer la déformation élastique de ladite plaque (2) dans une plage angulaire donnée.

## Description

L'invention concerne les dispositifs d'ostéosynthèse pour colonne vertébrale.

Dans la chirurgie de la colonne vertébrale, on utilise des dispositifs mécaniques pour relier deux ou plusieurs vertèbres consécutives. Cette chirurgie est pratiquée notamment en cas de rupture de vertèbre par suite d'accident ou en cas de dégénérescence des os des vertèbres ou des disques intervertébraux. Ces dispositifs mécaniques sont destinés à immobiliser ces vertèbres entre elles et à reprendre au moins en partie les efforts supportés habituellement par la colonne vertébrale. Ces moyens mécaniques comportent des organes d'ancrage fixés dans les vertèbres et un organe de liaison reliant les organes d'ancrage.

En général, l'organe de liaison est constitué d'une tige rigide. Cette disposition nécessite un alignement précis des têtes des organes d'ancrage. En outre, cette rigidité excessive peut entraîner des risques de dégénérescence du disque qui se trouve immédiatement au-dessus de l'ensemble des vertèbres appareillées et des contraintes mécaniques importantes sur l'ensemble restant de la colonne vertébrale.

Il a été en outre constaté qu'il est nécessaire de maintenir un certain degré de liberté entre deux vertèbres consécutives appareillées pour éviter une dégénérescence du disque intervertébral correspondant.

L'état de la technique est illustré notamment par WO 98/22033 qui prévoit, en tant qu'organe de liaison, une tige ayant deux portions d'extrémité rectilignes s'engageant dans des orifices prévus aux extrémités externes des moyens d'ancrage, et une portion médiane en forme d'anneau ouvert qui permet un certain degré de liberté autour de plusieurs axes de rotation. De par sa forme, cette pièce de liaison est difficile à réaliser.

EP 0 625 337 prévoit en tant qu'organe de liaison une coulisse en U qui présente une rainure médiane traversée par les organes d'ancrage, la coulisse passant à travers des bagues servant à immobiliser les moyens d'ancrage.

EP 0 820 730, qui représente l'état de la technique le plus proche de l'invention, concerne une plaque d'ostéosynthèse dont la flexibilité varie le long de sa longueur. A cet effet, l'épaisseur de la plaque décroît d'une extrémité à l'autre, et sa souplesse peut être augmentée grâce à des encoches sous forme de rainures semi-cylindriques, positionnées entre deux perçages successifs tronconiques destinés à recevoir une tête de vis de fixation.

Le but de l'invention est de proposer un système d'ostéosynthèse pour colonne vertébrale qui soit facile à réaliser et facile à mettre en oeuvre.

L'invention concerne donc un dispositif d'ostéosynthèse pour colonne vertébrale, comportant au moins deux organes d'ancrage adaptés à être fixés à deux vertèbres consécutives de la colonne vertébrale, et un organe de liaison adapté à relier les deux organes d'ancrage en exerçant des contraintes à l'encontre du rapprochement des deux organes d'ancrage en translation l'un vers l'autre, l'organe de liaison étant déformable élastiquement en flexion autour d'au moins un axe de déformation.

Le dispositif selon l'invention est caractérisé par le fait que l'organe de liaison est constituée d'une plaque courbée dans le sens antéro-postérieur pour s'adapter à la courbure de la colonne vertébrale et comporte des moyens d'immobilisation de chaque organe d'ancrage et par le fait que ladite plaque présente au moins sur l'une de ses faces une fente transversale rectiligne susceptible d'assurer la déformation élastique de ladite plaque dans une plage angulaire donnée.

Selon une première variante de réalisation de l'invention, la plaque comporte plusieurs fentes parallèles ménagées alternativement sur ladite face et la face opposée. Ces fentes ont de préférence des profondeurs supérieures à la moitié de l'épaisseur de la plaque.

Selon un mode de réalisation préféré, la plaque comporte deux fentes disposées dans le même plan transversal et ménagées respectivement dans ladite face et la face opposée de la plaque. Très avantageusement, les fentes débouchent chacune dans une cavité transversale et les deux cavités transversales sont séparées par une paroi mince qui s'étend dans un plan parallèle auxdites faces. Ce plan est de préférence le plan médian de la plaque.

Afin d'assurer une flexion dans n'importe quelle direction, la plaque comporte deux jeux de fentes, les fentes du deuxième jeu s'étendant dans une direction perpendiculaire aux fentes du premier jeu.

Avantageusement, les organes d'ancrage sont des vis pédiculaires dont les têtes ont des portées sphériques, et les moyens d'immobilisation d'une vis comportent un logement traversant ménagé dans la plaque, ce logement présentant une portée sphérique complémentaire de la portée sphérique de la tête de vis, et un filetage pour recevoir un cabochon à portée sphérique destiné à recouvrir et serrer la tête de vis.

Selon une autre caractéristique avantageuse, la plaque comporte au moins un logement traversant oblong permettant d'immobiliser la tête de vis dans une position choisie parmi plusieurs positions.

D'autres avantages et caractéristiques de l'invention ressortiront à la lecture de la description suivante faite à titre d'exemple et en référence aux dessins annexés dans lesquels :
- la figure 1 est une vue en coupe selon un plan vertical médian d'un dispositif d'ostéosynthèse selon l'invention ayant trois organes d'ancrage ;
- la figure 2 est une vue de dessus de la plaque du dispositif de la figure 1 ;
- la figure 3 est une vue en coupe transversale selon la ligne III-III de la figure 1 de la plaque et d'une vis pédiculaire ;
- la figure 4 est une vue de dessus d'une plaque seule destinée à relier deux vis pédiculaires ;
- la figure 5 est une vue latérale de la plaque de la figure 4 ;
- la figure 6, semblable à la figure 1, montre les positions extrêmes que peut prendre la vis médiane dans le plan vertical médian du dispositif ;
- la figure 7 semblable à la figure 3 montre les positions extrêmes que peut prendre la vis médiane dans un plan transversal de la plaque ;
- les figures 8a à 8d montrent différentes dispositions des fentes ménagées dans la plaque ; et
- la figure 9 montre à plus grande échelle une vue latérale de la plaque du dispositif selon un mode de réalisation préféré de l'invention dans une région comportant des fentes.

Sur les dessins, on a représenté par la référence 1 un dispositif d'ostéosynthèse pour colonne vertébrale qui comporte une plaque 2 courbée dans le sens antéro-postérieur afin qu'elle s'adapte à la courbure de la colonne vertébrale, des vis pédiculaires 3 dont les têtes 4 sont immobilisées, après appareillage d'un patient, dans des logements 8 ménagés dans la plaque 2, au moyen de cabochons 6.

Chaque vis pédiculaire 3 est autoforeuse et comporte une tête 4 sphérique présentant à son extrémité un logement 5 délimité par des empreintes femelles destinées à coopérer avec des empreintes mâles d'un outil de vissage.

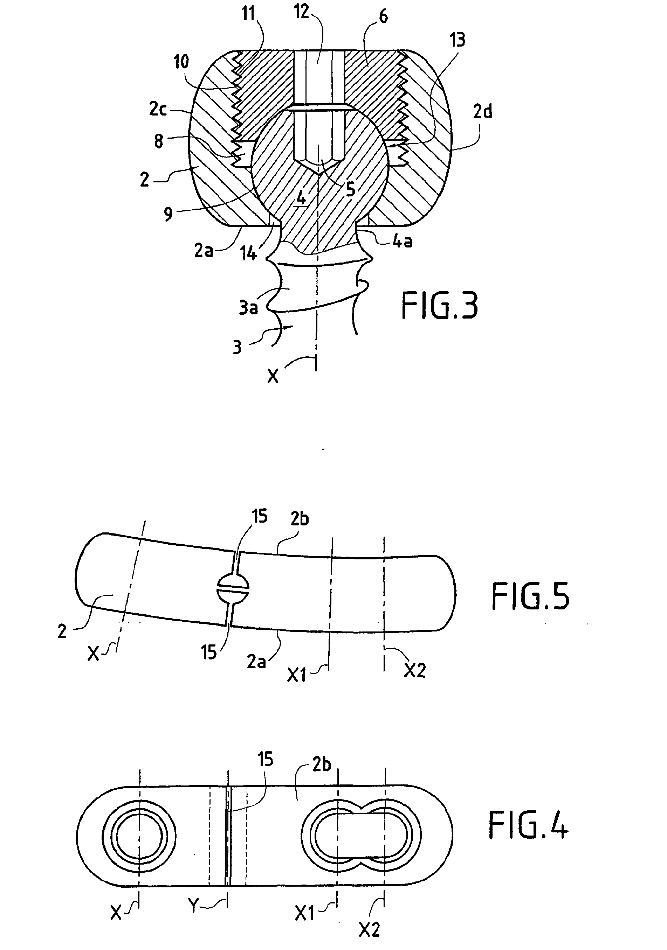

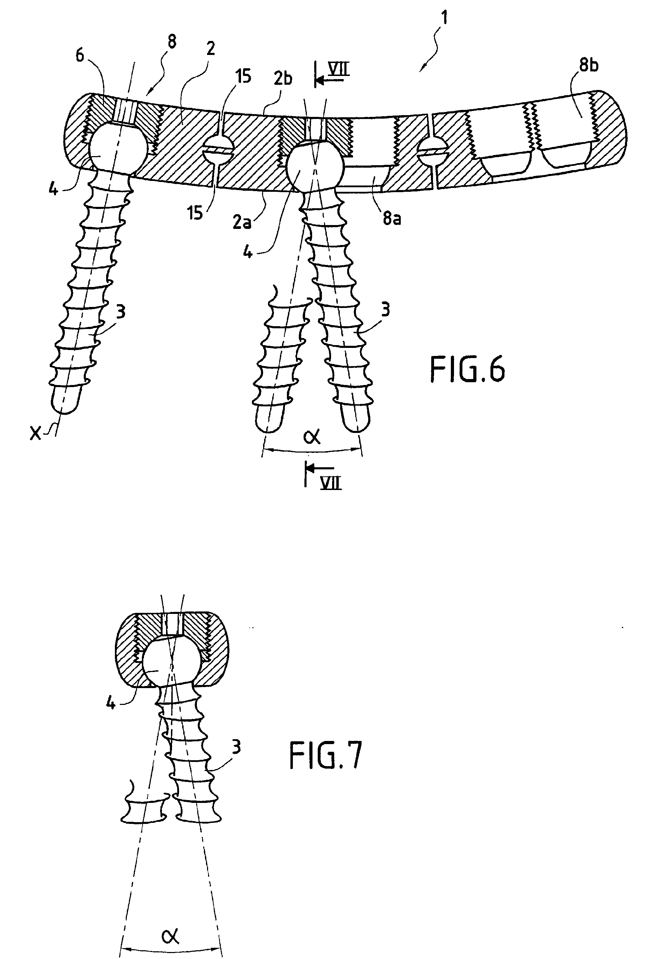

Ainsi que cela se voit sur les figures 3 et 7, les logements 8 de la plaque 2 traversent cette dernière. Chaque logement 8 est délimité du côté de la face inférieure convexe 2a de la plaque 2 par une portée sphérique 9 sur laquelle vient appuyer la base de la tête sphérique 4 et du côté de la face extérieure concave 2b de la plaque 2 par une paroi cylindrique filetée 10 ayant un diamètre au moins égal au diamètre de la tête de vis 4.

Le filetage de la paroi cylindrique 10 est destiné à coopérer avec un filetage 11 de la paroi périphérique d'un cabochon 6. Chaque cabochon 6, de forme annulaire, présente également un logement 12 délimité par des empreintes femelles destinées à coopérer avec les empreintes mâles d'un outil de serrage. Ce cabochon 6 présente également sur sa face inférieure une portée sphérique 13 destinée à venir en appui sur la tête 4 de la vis correspondante.

L'orifice 14 du logement 8 du côté de la face inférieure convexe 2a de la plaque 2 a un diamètre légèrement supérieur au diamètre de la zone de raccordement 4a de la tête de vis 4 avec la partie filetée 3a de la vis 3. Cette disposition permet un débattement d'un angle α de la vis 3 par rapport à la plaque 2, ainsi que cela est visible sur les figures 6 et 7.

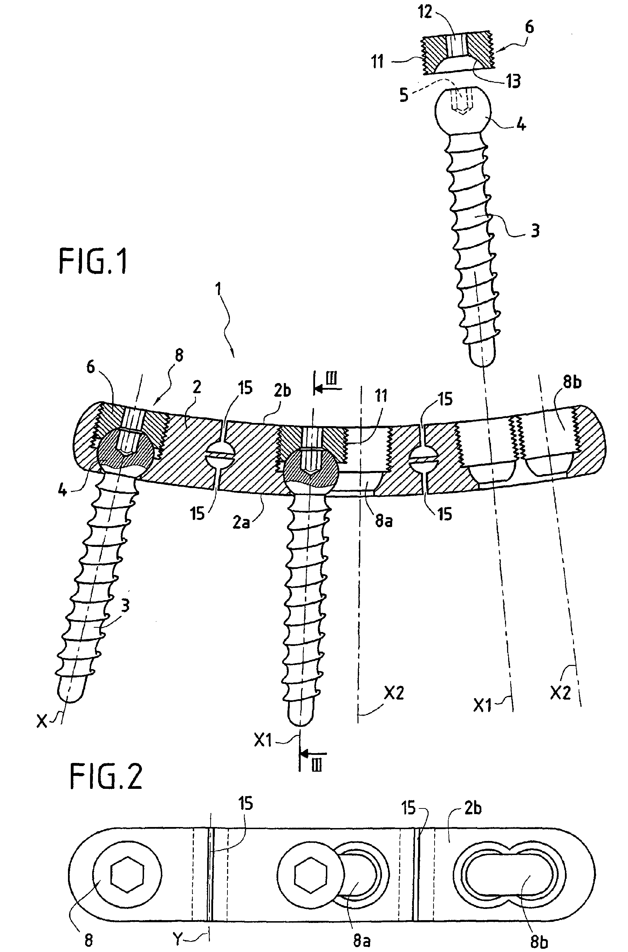

Ainsi que cela est montré sur la figure 1, la plaque 2 comporte à l'une de ses extrémités, un logement simple 8 de révolution autour d'un axe X, tandis que les deux autres logements 8a et 8b sont oblongs et sont réalisés par usinage d'au moins deux logements simples 8, sécants d'axes respectifs X₁ et X₂.

Dans ces logements oblongs 8a et 8b, la vis pédiculaire 3 correspondante peut se placer dans des positions proches.

Chaque vis pédiculaire 3 est destinée à être ancrée dans une vertèbre. Après avoir pris ses repères, le praticien positionne la plaque 2 et met en place une vis 3 à travers le logement 8. Il fait de même pour les deux autres vis pédiculaires 3 qui passent à travers les logements oblongs 8a et 8b.

Après avoir serré les trois vis pédiculaires 3, il immobilise les têtes 4 de ces vis au moyen des cabochons 6.

La plaque 2 et les vis 3 sont réalisées à partir d'un alliage de titane ou de tout autre matériau métallique biocompatible.

Pour donner à la plaque 2 un certain degré de flexion autour d'un axe transversal Y sensiblement perpendiculaire aux axes des vis pédiculaires 3, la plaque 2 présente entre deux logements adjacents 8, 8a et 8a, 8b, au moins une fente transversale 15 qui débouche sur les faces latérales 2c, 2d de la plaque 2.

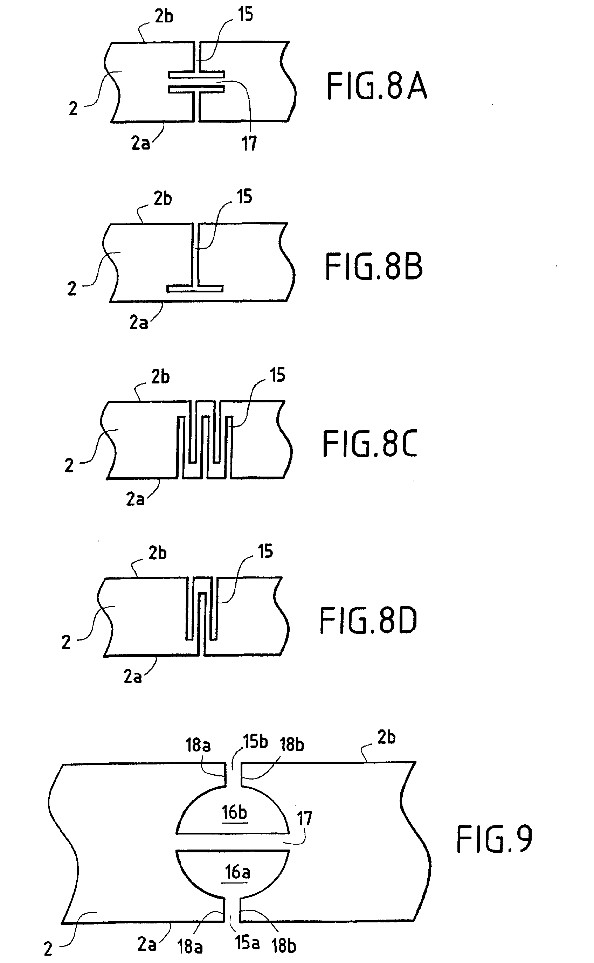

De préférence, ainsi que cela est montré sur la figure 9, la plaque 2 comporte entre deux logements consécutifs, deux fentes opposées 15a, 15b ménagées respectivement dans la face inférieure concave 2a et la face supérieure convexe 2b de la plaque 2. Ces fentes 15a et 15b débouchent à l'intérieur de la plaque 2, respectivement dans des cavités transversales 16a, 16b séparées par une paroi mince 17 qui s'étend parallèlement aux faces 2a et 2b de la plaque 2. L'épaisseur de la paroi mince 17 est calculée pour donner à la plaque 2 l'élasticité voulue autour de l'axe transversal Y. L'angle de flexion de la plaque 2 est limité par l'épaisseur des fentes 15a et 15b. Lorsque les bords 18a, 18b d'une fente 15a ou 15b viennent en appui l'un contre l'autre, la plaque 2 ne peut fléchir davantage.

Les figures 8a à 8d montrent diverses possibilités de réalisation des fentes 15 pour assurer l'élasticité de la plaque 2.

La figure 8a est semblable au mode de réalisation de la figure 9, si ce n'est que les cavités 16a et 16b sont remplacées par des fentes parallèles aux faces 2a et 2b de la plaque 2 et séparées par une paroi mince 17.

La figure 8b montre un mode de réalisation dans lequel une seule fente 15 est réalisée à partir de la face supérieure concave 2b. Cette fente 15 est relativement profonde et limite la flexion de la plaque 2 dans une seule direction.

Les figures 8c et 8d montrent un mode de réalisation dans lequel la plaque 2 comporte plusieurs fentes 15 parallèles ménagées alternativement sur la face inférieure convexe 2a et la face supérieure concave. Ces fentes ont de préférence une profondeur supérieure à la moitié de l'épaisseur de la plaque 2.

Ainsi que cela est montré sur la figure 3, les faces latérales 2c, 2d de la plaque 2 sont concaves.

Il est à noter que ces faces 2c et 2d peuvent être planes, et que l'on peut ménager sur ces faces 2c, 2d des deuxièmes fentes perpendiculaires à la direction des fentes 15 décrites précédemment, et qui autoriseraient une flexion de la plaque 2 autour d'un deuxième axe perpendiculaire à l'axe Y et sensiblement parallèle aux axes des vis 3..

La réalisation de la plaque 2 avec ses logements 8, 8a, 8b et ses fentes 15 ne pose pas de problème particulier.

La description détaillée faite ci-dessus en référence aux figures 1 et 6, concerne une plaque 2 ayant trois logements 8, 8a et 8b et qui permet de relier entre elles trois vertèbres consécutives d'un patient.

Il est évident que l'invention se rapporte également à un dispositif pour relier deux vertèbres consécutives, tel que celui montré sur les figures 4 et 5, dans lequel la plaque 2 comporte un logement simple 8 et un seul logement oblong 8a.

## Revendications

1. Dispositif d'ostéosynthèse pour colonne vertébrale, comportant au moins deux organes d'ancrage (3) adaptés à être fixés à deux vertèbres consécutives de la colonne vertébrale, et un organe de liaison (2) adapté à relier les deux organes d'ancrage (3) en exerçant des contraintes à l'encontre du rapprochement des deux organes d'ancrage (3) en translation l'un vers l'autre, l'organe de liaison (2) étant déformable élastiquement en flexion autour d'au moins un axe de déformation,
**caractérisé par le fait que** l'organe de liaison est constitué d'une plaque (2) courbée dans le sens antéro-postérieur pour s'adapter à la courbure de la colonne vertébrale, et comporte des moyens d'immobilisation (6, 8) de chaque organe d'ancrage (3), et
**par le fait que** ladite plaque (2) présente entre deux moyens d'immobilisation, au moins une fente (15) transversale susceptible d'assurer la déformation élastique de ladite plaque (2) dans une plage angulaire donnée.

2. Dispositif selon la revendication 1, **caractérisé par le fait que** la plaque (2) comporte plusieurs fentes parallèles (15) ménagées alternativement sur une face (2a) et la face opposée (2b) de la plaque (2).

3. Dispositif selon la revendication 2, **caractérisé par le fait que** les fentes (15) ont des profondeurs supérieures à la moitié de l'épaisseur de la plaque (2).

4. Dispositif selon la revendication 1, **caractérisé par le fait que** la plaque comporte deux fentes (15a, 15b) disposées dans le même plan transversal, ménagées respectivement dans une face (2a) et la face opposée (2b) de la plaque (2).

5. Dispositif selon la revendication 4, **caractérisé par le fait que** les fentes (15a, 15b) débouchent chacune dans une cavité transversale (16a, 16b), les deux cavités (16a, 16b) étant séparées par une paroi mince (17) qui s'étend dans un plan parallèle auxdites faces (2a, 2b).

6. Dispositif selon la revendication 5, **caractérisé par le fait que** la paroi mince (17) est située dans le plan médian de la plaque (2).

7. Dispositif selon l'une quelconque des revendications 1 à 6, **caractérisé par le fait que** la plaque (2) comporte deux jeux de fentes, les fentes du deuxième jeu s'étendant dans une direction perpendiculaire aux fentes du premier jeu.

8. Dispositif selon l'une quelconque des revendications 1 à 7, **caractérisé par le fait que** les organes d'ancrage (3) sont des vis pédiculaires dont les têtes (4) ont des portées sphériques, et **par le fait que** les moyens d'immobilisation d'une vis (3) comportent un logement traversant (8) ménagé dans la plaque (2), ce logement (8) présentant une portée sphérique (9) complémentaire de la portée sphérique de la tête de vis (4), et un filetage (10) pour recevoir un cabochon (6) à portée sphérique destiné à recouvrir et serrer la tête de vis (4) dans son logement.

9. Dispositif selon la revendication 8, **caractérisé par le fait que** la plaque (2) comporte au moins un logement traversant (8a, 8b) oblong permettant d'immobiliser la tête de vis (4) dans une position choisie parmi plusieurs positions.
